# EUROPEAN PATENT APPLICATION

(11) **EP 4 483 893 A1**
(43) Date of publication of application: **01.01.2025**
(21) Application number: 23759735.6
(22) Date of filing: 10.02.2023
(51) Int. Cl.: A61K 39/00, A01K 61/13

(54) **VACCINATION METHOD AND FARMED FISH**

(30) Priority: 22.02.2022 JP 2022025988
(71) Applicant: Nissui Corporation, Tokyo 105-8676 (JP)
(72) Inventor: UMEDA, Naoko, Hachioji-shi, Tokyo 192-0991 (JP); HARA, Takashi, Karatsu-City, Saga 8470192 (JP); MIYAKE, Toshimune, Karatsu-City, Saga 8470192 (JP); UMEDA, Akira, Karatsu-City, Saga 8470192 (JP)
(74) Representative: Mewburn Ellis LLP
(86) International application number: PCT/JP2023/004653
(87) International publication number: WO 2023/162731

(57) **Abstract**

A vaccination method including inoculating a vaccine liquid by inserting an injection needle into a back or tail muscle of farmed fish.

## Description

### Technical Field

The present invention relates to a method of vaccinating a farmed fish and to a vaccinated farmed fish.

### Background Art

In tuna farming, damage of Streptococcosis and Iridovirus disease is spreading. At present, there is no method of preventing Streptococcosis or Iridovirus disease in farmed tuna other than vaccines. Vaccines against fish Iridovirus infections are described in JP 2019-119732 A. Generally, a fish is vaccinated by injecting a vaccine liquid into the abdominal cavity of an anesthetized fish, and intramuscular injection is approved only for some vaccines such as red sea bream vaccines and some fish species. "Streptococcosis occurring in farmed bluefin tuna" authored by Yuji Ishii (Letter from Fisheries Research Center (Cultivation Resource Research Institute, Fisheries Research Center, Ehime Research Institute of Agriculture, Forestry and Fisheries), No. 11, pp. 6-7 (January 2019)) describes measures against Streptococcosis in bluefin tuna, but describes that there is a demerit in the risk of death due to scratching of the body surface caused by handling, and that it is considered difficult to vaccinate by injection.

### SUMMARY OF INVENTION

### Technical Problem

In the case of farmed fish such as yellowtail, vaccination is usually carried out by intraperitoneal administration. However, when vaccination is carried out by intraperitoneal administration to a fish species, such as tuna, whose organs reach the wall of the abdominal cavity, the organs may be injured by a needle at the time of inoculation. Further, since tuna is largely worn due to handling and anesthesia, a general intraperitoneal vaccination method cannot be adopted. On the other hand, intramuscular injection is relatively easy to handle, but the vaccine remains at an injected muscle site or forms a granuloma, and thus the commercial value of an edible part may be lost.

In view of the above problems, the present application provides, for farmed fish such as tuna, a vaccination method that minimizes handling stress and also minimizes the loss of a commercial value of an edible part.

### Solution to Problem

### (1) Vaccination Method

A vaccination method according to a first embodiment of the present application includes inoculating a vaccine liquid by inserting an injection needle into a back muscle or a tail muscle of a farmed fish.

A position to insert the injection needle is desirably a site along a dorsal midline of the farmed fish. In addition, the position to insert the injection needle is desirably a dorsal fin base. Further, the farmed fish is desirably unanesthetized. An adjuvant is desirably added to the vaccine liquid.

The farmed fish is desirably a fish species of the family Scombridae, especially tuna species or bonito species.

### (2) Farmed Fish

A farmed fish according to a second embodiment of the present application is characterized by having a trace of vaccination on the back or the tail. The farmed fish desirably has the trace at a site along its dorsal midline, especially at its dorsal fin base.

The farmed fish is desirably a fish species of the family Scombridae, especially tuna species or bonito species.

### Advantageous Effects of Invention

Since the embodiments of the present application are configured as described above, it is possible to provide, for farmed fish such as tuna, a vaccination method that minimizes handling stress and also minimizes the loss of a commercial value of an edible part.

### Brief Description of Drawing

FIG. 1 is a plan view schematically illustrating each site of a farmed fish.
FIG. 2 is a graph indicating the transition of cumulative mortality rates of farmed fishes after administration of a vaccine of a comparative example.

### DESCRIPTION OF EMBODIMENTS

Farming equipment that performs the vaccination method of the present embodiment is not particularly limited, and may be appropriately selected in consideration of the kind, size, and the like, of the target farmed fish. However, it is necessary to ensure a sufficient size to avoid collision between the farmed fishes immediately after vaccination or between the farmed fish and the farming equipment. For example, in the case of bluefin tuna, for example, a square fish preserve is desirably 20 m or more in both length and width and 15 m or more in depth, and a round fish preserve is desirably 20 m or more in diameter and 15 m or more in depth. In the case of the square fish preserve, the upper limit is desirably 100 m or less in both length and width and 50 m or less in depth, and in the case of the circular fish preserve, the upper limit is desirably 100 m or less in diameter and 50 m or less in depth. This is because workability is deteriorated above the upper limits.

The vaccination method of the present embodiment can be carried out in either marine aquaculture facility or land-based aquaculture facility. In the case of the marine aquaculture facility, farming can be continued with no change. On the other hand, in the land-based aquaculture facility, it is easy to observe the influence of the vaccine after vaccination.

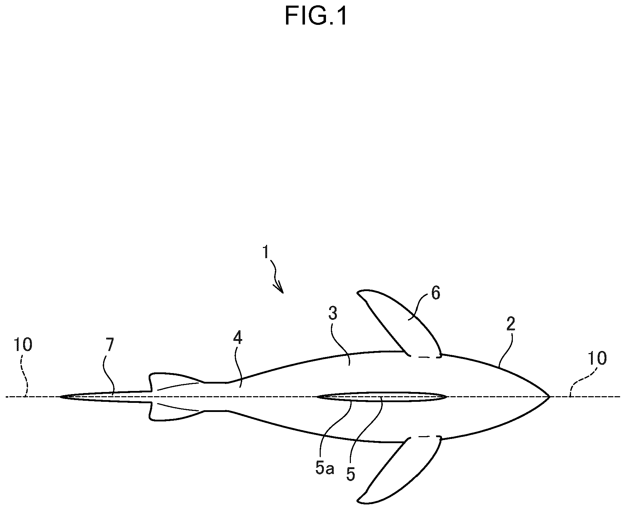

The vaccination method of the present embodiment is suitable for fish species of the family Scombridae having low resistance to handling stress, particularly tuna species or bonito species. An example of the farmed fish is illustrated in the plan view of FIG. 1. Farmed fish 1 in this figure is tuna. Farmed fish 1 of this example has a spindle shape in plan view, and a pair of pectoral fins 6 are present at a base of a head 2. A tail fin 7 is present at a tip of a tail 4. A dorsal fin 5 is present along a midline 10 on a back 3.

The vaccination method of the present embodiment can be applied to farmed fish having an arbitrary size, but may be applied to farmed fish having a size in a range in which it can be caught. However, if the farmed fish is too large, a series of vaccinations, including picking up from a fish preserve and fixing at the time of vaccination, may not be efficiently performed. For example, in the case of a bluefin tuna, when the fish weight is 6.0 kg or less, desirably 4.0 kg or less, and more desirably 2.0 kg or less, the tuna can be vaccinated without applying excessive stress to the tuna. The farmed fish using the vaccination method of the present embodiment may have developed immunity to such an extent that an antibody can be produced upon vaccination. For example, in the case of a bluefin tuna, the vaccination method may be carried out using fish having a fish weight of 0.3 kg or more, desirably 0.4 kg or more, more desirably 0.5 kg or more.

After scooped up, the farmed fish may be firmly fixed not to move during vaccination. For example, the farmed fish may be fixed on a floor or a table by hand or using an instrument. Since it is necessary to return the farmed fish into the fish preserve immediately after vaccination, it is convenient to fix it by the operator's hand. Since the farmed fish in the present embodiment is vulnerable to rubbing, the farmed fish is preferably fixed to avoid rubbing. In the case of fixing the farmed fish by the operator's hand, for example, a rubber glove may be used to avoid direct touch.

When the farmed fish is fixed on a floor or a table, a cushion is laid on the floor or the table, and the farmed fish is placed on the cushion, the farmed fish can be pressed down strongly to some extent because the cushion cushions the farmed fish. When the farmed fish is fixed, the farmed fish is preferably not touched with a material causing rubbing. This is because if rubbing can occur, it may later die from bacterial infection. For example, the farmed fish may be fixed with a material that does not cause rubbing, such as a vinyl bag or a vinyl sheet, even when touching the farmed fish.

When vaccinated, the farmed fish is desirably unanesthetized. In general, when farmed fish is anesthetized, the fish is caught or scooped up from a fish preserve, immersed in an anesthetic bath containing an anesthetic liquid diluted with seawater, and then scooped up from the anesthetic bath. This increases a chance of touch with the fish during anesthesia. However, in the case of tuna species, mucus and scales drop off due to touch with its body surface, causing an abrasion of the skin, from which bacterial infection tends to occur, leading to a decrease in productivity. Thus, vaccination without anesthesia can reduce the chance of touch to tuna species.

The vaccination method according to the present embodiment includes inoculating a vaccine liquid by inserting an injection needle into a back or tail muscle of farmed fish. As used herein, the term "back or tail muscle" refers to a muscle around the pterygiophore of the back (or between split parts) in a region along the dorsal midline, or a muscle on a back side near the head or in an edible part at a site with relatively low utility in the tail.

For minimizing the loss of the commercial value of the edible part, the position to insert the injection needle is desirably a site along the dorsal midline of the farmed fish, especially a site that is to be discarded when the farmed fish is sectioned for eating. In other words, the position to insert the injection needle during the vaccination according to the present embodiment is desirably a muscle at a site along the midline 10 in the back 3. This is because, by using the midline 10 as a marking, it becomes easy to perform inoculation while avoiding the muscle that is usually an edible part, and it is also possible to remove a site with a possibility that the vaccine may remain from the edible part.

In addition, the position to insert the injection needle is desirably a dorsal fin base. The dorsal fin base has many bone and skin parts, and when the vaccine is inoculated into this site, it is discarded when the fish is sectioned for eating, and thus the loss of the commercial value of the farmed fish 1 can be minimized.

A depth to which the injection needle is inserted during vaccination is desirably 3 mm or more and less than 1.0 cm. If the depth is 1.0 cm or more, the inside of the body may be injured, and if the depth is less than 3 mm, the inoculated vaccine may leak. For example, an injector having a needle of about 5 mm is preferably used for vaccination because the entire length of the needle can be inserted. A thickness of the injection needle can be appropriately selected depending on the size of the farmed fish, and is preferably from 21 to 23 gauge, for example. However, if the weight of the farmed fish is about 500 g, the lower limit of the depth may be 1 mm. If the weight of the farmed fish is 6 kg or more, the upper limit of the depth may be less than 1.5 cm. In addition, the vaccination of the present embodiment may be carried out using a continuous injector when the vaccination is carried out continuously for a plurality of fish. When the vaccine is inoculated using the continuous injector, the vaccine can be continuously administered without filling the vaccine in the middle of the injection, thereby simplifying the operation.

Examples of diseases as targets for vaccination in the present embodiment include, in addition to Streptococcosis and Iridovirus disease, vibriosis, pseudotuberculosis of yellowtail, pasteurellosis of tuna, and nocardiosis. Vaccines against the respective diseases may be used as a mixture, or may be used as separate vaccines. The vaccines are used in combination, thereby making it possible to reduce the number of vaccinations.

An adjuvant is desirably added to the vaccine liquid to enhance the antibody production effect. As the adjuvant, an aluminum gel or an oil adjuvant or a combination thereof can be used. The use of the adjuvant can induce a high antibody titer or increase the duration of antibody induction, or both. The oil adjuvant is preferred because it can be expected to induce a higher antibody titer and a longer duration of antibody induction than the aluminum gel.

The water temperature at the time of vaccination in the present embodiment can be appropriately determined depending on the target farmed fish and the target disease. For example, the water temperature when the bluefin tuna is vaccinated against Streptococcosis and Iridoviruses is 14°C or higher and 30°C or lower, desirably 20°C or higher and 28°C or lower, more desirably 24°C or higher and 28°C or lower.

The vaccination of the present embodiment is desirably performed at a stage where the target fish disease has not occurred in the same water area. This is because fish may suffer from the disease before the antibody titer sufficiently increases.

When feed is present in the digestive tract of the farmed fish, oxygen deficiency tends to occur during the vaccination operation, and thus it is desirable to stop feeding at least one day before vaccination. In addition, since tuna cannot sufficiently breathe unless it swims, it cannot breathe while it is pulled up from water. Therefore, for reducing stress given to the farmed fish, it is preferable to return the fish into the fish preserve immediately after vaccination.

The time required for a series of operations from scooping up of the farmed fish through vaccination to returning thereof to the fish preserve is preferably as short as possible. For example, in the case of a bluefin tuna, the operation time is preferably within one minute. A place for vaccination may be anywhere as long as it is close to the fish preserve. Vaccination is desirably performed, for example, on a ship or on a raft in view of the possibility of returning the fish into the fish preserve immediately after vaccination.

Feeding of the vaccinated farmed fish may be restricted for several days or may be performed without any special restriction on feeding. Normally, when vaccinated, poor feeding occurs in yellowtail or greater amberjack, but vaccination by the method described in the present embodiment may not cause poor feeding, and enables normal feeding. This makes it possible to avoid the time loss for rearing of the farmed fish to a big size.

In the farmed fish inoculated with the vaccine by the vaccination method of the present embodiment, a trace of vaccination, such as scar contraction or swelling, may be observed on its back or tail. In this case, it can be easily determined whether or not the vaccine has been inoculated by the vaccination method of the present embodiment. For example, if the vaccine is inoculated at a site along the dorsal midline, a trace will be seen at that site. Also, if the vaccine is inoculated at a dorsal fin base, a trace will be seen at that site. The trace can be determined with the naked eye or using a microscope. Such a trace of vaccination may be used as a marking for a portion that should be discarded at the time of sectioning.

According to the vaccination method of the present embodiment, even farmed fish such as tuna species, which is not suitable for intraperitoneal inoculation, can be vaccinated relatively easily and with minimum handling stress, and the loss of the commercial value of the edible part due to vaccination can be minimized.

The number of times of vaccination in the present embodiment may be one or more. A second or subsequent booster inoculation may be administered after a predetermined period of time, such as 2 weeks, 1 month, 2 months, 6 months or 1 year. In addition, an oral vaccine may be used in combination as a booster inoculation in consideration of handling stress due to multiple vaccinations without anesthesia.

### EXAMPLES

The vaccination method of the present application will be described in the following Examples.

### (1) Test Method

At the Goto Office of Kaneko Sangyo Co., Ltd., Goto City, Nagasaki Prefecture, young fishes of bluefin tuna having a fish weight of from 0.2 to 0.5 kg were inoculated with a vaccine at any time upon arrival, and then housed in a fish preserve. "Bicipack Injection 5 Oil" manufactured and sold by Kyoritsu Seiyaku Corporation was used as the vaccine and inoculated into the dorsal fin base, avoiding general edible parts. The inoculation was carried out between August 2, 2021 and August 5, 2021, as needed, and the total number of inoculated fish was 120 eventually. The water temperature at the time of vaccination was about 27°C.

Here, the target diseases of Bicipack 5 Oil used as the vaccine are type I Streptococci, type II Streptococci, Iridoviruses, Vibrios and Pasteurella piscicida. The main components of the oil adjuvant contained in the vaccine are ethyl oleate and squalane. When the present vaccine is administered to yellowtail, the dosage regimen of the present vaccine is determined as follows: 0.1 ml of the vaccine is administered once into the abdominal cavity of yellowtail having a weight of from 20 g to 1 kg using a continuous injector. However, in the present example, 0.2 ml was administered per one young Pacific bluefin tuna (Yokowa).

As a comparative example, a case was observed in which, in 2007, a mixed inactivated vaccine "BIKEN" against Iridoviruses and Streptococci, manufactured by The Research Foundation for Microbial Diseases of Osaka University and sold by Dainippon Pharma Co., Ltd., was intraperitoneally administered to the same fish species. At the time of the vaccination, the fishes were divided into three groups: a first group in which they were anesthetized, a second group in which they were retained on a sponge mat without anesthesia, and a third group in which they were retained in a plastic bag without anesthesia. The water temperature at the time of vaccination was about 28°C. The cumulative mortality rate after the vaccination was then calculated by the following formula: Cumulative mortality rate (%) = [number of dead tuna during observation period]/[number of tuna at start of observation] × 100

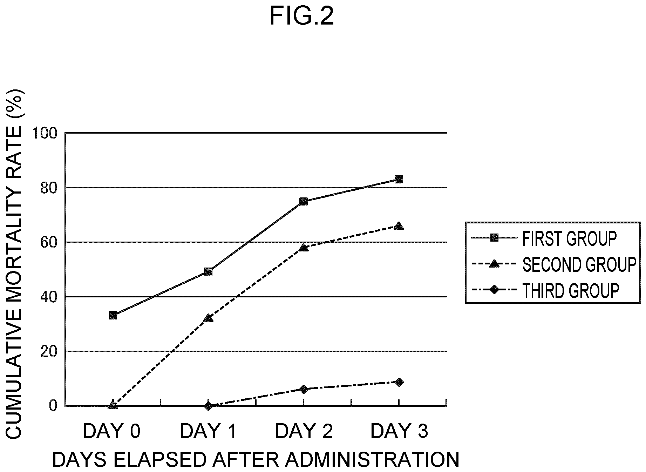

FIG. 2 and the following Table 1 indicates the transition of the cumulative mortality rates in the above-stated comparative example.

**[Table 1]**

| Cumulative mortality rate (%) | | | | |
|---|---|---|---|---|
| Days after administration | 0 | 1 | 2 | 3 |
| First group | 34 | 49 | 75 | 83 |
| Second group | 0 | 33 | 57 | 65 |
| Third group | 0 | 0 | 7 | 10 |

At the time of the vaccination, the fishes were divided into three groups: a first group in which they were anesthetized, a second group in which they were retained on a sponge mat without anesthesia, and a third group in which they were retained in a plastic bag without anesthesia. As a result, three days after the administration, the cumulative mortality rate was 83% for the first group indicated by the solid line in FIG. 2, the cumulative mortality rate was 65% for the second group indicated by the broken line, and the cumulative mortality rate was 10% even for the third group indicated by the dotted line and having the lowest cumulative mortality rate. It is presumed that the retention on the sponge mat resulted in a relatively high cumulative mortality rate due to damage caused by scratching the surfaces of the farmed fishes, whereas the retention in the vinyl bag which does not cause scratching of the surfaces of the farmed fishes as much as possible resulted in the lowest cumulative mortality rate.

On the other hand, the cumulative mortality rate one month after the vaccination in this example was 3.3% for the inoculated group and 6.2% in the non-inoculated group. In the inoculated group, the decrease in feeding activity observed when the present vaccine was intraperitoneally inoculated was not observed.

In the example, one month after the vaccination, blood was collected from five fish of each of the non-inoculated group and the inoculated group, and measured for the antibody titer against the Streptococcus, type I Lactococcus garvieae, by a microtiter method. The results are presented in Table 2. In the geometric mean calculation, when the antibody titer was less than 2, the antibody titer was defined as 1.

**[Table 2]**

| Identification No. | Non-inoculated group | Inoculated group |
|---|---|---|
| 1 | <2 | 16 |
| 2 | <2 | 256 |
| 3 | 32 | 32 |
| 4 | 2 | 2048 |
| 5 | 2 | 32 |
| Geometric mean | 2.64 | 97.0 |

As the above result, it was found that the antibody titer against the Streptococcus in the inoculated group was significantly higher than that in the non-inoculated group. No death due to Streptococcosis was observed in both the inoculated group and the inoculated group.

Similarly, the antibody titer against the Iridovirus was measured by an ELISA-method. The results are presented in Table 3 below.

**[Table 3]**

| Identification No. | Non-inoculated group | Inoculated group |
|---|---|---|
| 1 | 0.159 | 0.694 |
| 2 | 0.225 | 0.292 |
| 3 | 0.462 | 0.498 |
| 4 | 0.419 | 0.657 |
| 5 | 0.311 | 0.516 |
| Arithmetic mean | 0.315 | 0.531 |

As the above result, it was found that the antibody titer against the Iridovirus in the inoculated group was significantly higher than that in the non-inoculated group. No death due to Iridovirus disease was observed in both the inoculated group and the inoculated group.

### Industrial Applicability

The present invention is applicable to vaccination of farmed fish.

## Claims

1. A vaccination method, comprising inoculating a vaccine liquid by inserting an injection needle into a back muscle or a tail muscle of a farmed fish.

2. The vaccination method according to claim 1, wherein a position to insert the injection needle is a site along a dorsal midline of the farmed fish.

3. The vaccination method according to claim 1, wherein a position to insert the injection needle is a dorsal fin base.

4. The vaccination method according to any one of claims 1 to 3, wherein the farmed fish is unanesthetized.

5. The vaccination method according to any one of claims 1 to 4, wherein an adjuvant is added to the vaccine liquid.

6. The vaccination method according to any one of claims 1 to 5, wherein the farmed fish is a fish species of the family Scombridae.

7. The vaccination method according to claim 6, wherein the fish species of the family Scombridae is tuna species or bonito species.

8. A farmed fish having a trace of vaccination on a back or a tail.

9. The farmed fish according to claim 8, having the trace at a site along a dorsal midline.

10. The farmed fish according to claim 8, having the trace at a dorsal fin base.

11. The farmed fish according to any one of claims 8 to 10, which is a fish species of the family Scombridae.

12. The farmed fish according to claim 11, wherein the fish species of the family Scombridae is tuna species or bonito species.
